# EUROPEAN PATENT APPLICATION

(11) **EP 4 851 616 A1**
(43) Date of publication of application: **09.12.2026**
(21) Application number: 25747961.8
(22) Date of filing: 26.01.2025
(51) Int. Cl.: G01N 27/30

(54) **BIOSENSOR AND ANALYTE MONITORING APPARATUS**

(30) Priority: 01.02.2024 CN 202410144883
(71) Applicant: SHENZHEN SISENSING CO., LTD., Shenzhen Guangdong 518110 (CN)
(72) Inventor: HAN, Xiongqi, Shenzhen, Guangdong 518110 (CN); FANG, Junfei, Shenzhen, Guangdong 518110 (CN); XIE, Fang, Shenzhen, Guangdong 518110 (CN)
(74) Representative: Vossius & Partner Patentanwälte Rechtsanwälte mbB
(86) International application number: PCT/CN2025/075253
(87) International publication number: WO 2025/162397

(57) **Abstract**

The present disclosure provides a biosensor and an analyte monitoring device, the biosensor including a first electrode provided with a sensing layer, and a polymer membrane covering at least the sensing layer and the first electrode, the polymer membrane comprising a first membrane layer and a second membrane layer, the first membrane layer comprising a substance that causes damage to the sensing layer and/or the first electrode upon direct contact, the second membrane layer being closer to the first electrode than the first membrane layer to separate the first membrane layer from the sensing layer and the first electrode. In accordance with the present disclosure, a biosensor and analyte monitoring device can be provided having a wider material selection range that can meet the requirements of more application scenarios.

## Description

### BACKGROUND OF INVENTION

The present disclosure generally relates to the field of new chemical materials. More specifically the present disclosure relates to a biosensor and an analyte monitoring device.

### Description of Related Art

Biosensors are analytical devices that combine biological materials, biologically derived materials, or biomimetic materials with optical, electrochemical, temperature, piezoelectric, magnetic, or micromechanical physico-chemical sensors or sensing microsystems, and are commonly used to rapidly detect certain specific chemical substances in the human body, such as glucose, ketone bodies, and uric acid.

Typically, in a biosensor, a sensing layer containing a specific sensitive substance for the analyte, such as enzymes, antibodies, and ligands, is disposed on the working electrode; during monitoring, the analyte reacts upon contact with the sensing layer to generate a signal, from which the analyte level can be determined. Moreover, in biosensors, a polymer membrane with various functionalities, such as a diffusion-limiting membrane, anti-interference membrane, or temperature-compensation membrane, is typically applied over the working electrode and the sensing layer.

However, in existing biosensors, to avoid damaging internal structures such as electrodes and the sensing layer, polymer membranes are generally selected from materials that are harmless or have minimal impact on electrodes and enzymes, thereby limiting material selection and performance optimization of functional layers.

### SUMMARY OF INVENTION

The present disclosure has been made in view of the above-described state of the art, and its objective is to provide a biosensor and analyte monitoring device with an expanded material selection range that may meet the demands of a broader range of applications.

To this end, a first aspect of the present disclosure provides a biosensor, the biosensor comprising a first electrode provided with a sensing layer, and a polymer membrane covering at least the sensing layer and the first electrode; the polymer membrane comprising a first membrane layer and a second membrane layer, the first membrane layer comprising a substance that causes damage to the sensing layer and/or the first electrode upon direct contact, the second membrane layer being located closer to the first electrode than the first membrane layer to separate the first membrane layer from the sensing layer and the first electrode.

In the first aspect of the present disclosure, the polymer membrane of the biosensor includes a second membrane layer and a first membrane layer, the first membrane layer comprising a substance harmful to the sensing layer and/or the first electrode; by incorporating the second membrane layer, the first membrane layer is separated from the internal structure (the sensing layer and the first electrode), thereby reducing adverse effects of the harmful substance in the first membrane layer on the sensing layer and the first electrode (e.g., corrosion leading to compromised structural integrity and functional loss). That is, in the present disclosure, a first membrane layer containing substances that have a negative impact on the electrode structure is innovatively selected, and the design of the second membrane layer resolves the performance bottleneck caused by limited material choices in conventional designs. This approach expands the material selection range for the functional membrane layer (first membrane layer), enhances the performance and multifunctionality of the biosensor, improves the stability and reliability of the sensor, and provides new ideas and possibilities for innovation and application expansion in biosensor technology. Thus, it is possible to provide a biosensor with a wider material selection range, which may be adapted to the requirements of more application scenarios.

In the biosensor according to the first aspect of the present disclosure, optionally, the first membrane layer is configured to reduce the influence of a sterilizing gas on the sensing layer when the biosensor is subjected to gas sterilization. In this case, the biosensor can be sterilized using a gas sterilization method, and compared to biosensors without the first membrane layer, the biosensor of the present disclosure exhibits a significantly reduced decrease in sensitivity before and after sterilization, and can maintain high sensitivity after sterilization. Thus, it is possible to provide a biosensor resistant to sterilizing gas.

In the biosensor according to the first aspect of the present disclosure, optionally, the first membrane layer is made from a first solution comprising a first-type polymer and a solvent.

In the biosensor according to the first aspect of the present disclosure, optionally, the first-type polymer is polyurethane. In this case, a first membrane layer comprising polyurethane may help limit the permeation of sterilizing gas during gas sterilization, forming a barrier to reduce the influence of the sterilizing gas on the sensing layer, particularly the influence of gaseous ethylene oxide on the active substance in the sensing layer, thereby allowing the biosensor to be sterilized using a gas sterilization method.

In the biosensor according to the first aspect of the present disclosure, optionally, the mass fraction of the first-type polymer in the first solution is 0.1% to 15%. Thus, the first membrane layer may help restrict the permeation of the sterilizing gas during gas sterilization.

In the biosensor according to the first aspect of the present disclosure, optionally, the solvent of the first solution may cause damage to the sensing layer and/or the first electrode upon direct contact. In this case, selecting a solvent with stronger solvating power capable of dissolving certain specific polymers (e.g., some non-polar or highly polar solvents can dissolve more types of polymers, including those difficult to process with conventional solvents) helps broaden the material selection range for polymers in the first membrane layer.

In the biosensor according to the first aspect of the present disclosure, optionally, the solvent of the first solution is an organic solvent, which is a mixed solvent of any one or more of tetrahydrofuran, dichloromethane, and N,N-dimethylacetamide. Thus, the first-type polymer can be dissolved using an organic solvent.

In the biosensor according to the first aspect of the present disclosure, optionally, the first membrane layer has a diffusion-limiting effect on the analyte sensed by the biosensor.

In the biosensor according to the first aspect of the present disclosure, optionally, the first membrane layer has biocompatibility.

In the biosensor according to the first aspect of the present disclosure, optionally, the first solution comprises a first-type polymer, the first-type polymer is polyurethane, the first-type polymer has a soft segment, and the proportion of the soft segment in the first-type polymer is 5% to 50%. Since the proportion of the soft segment affects the properties of the polyurethane material, in this case, selecting an appropriate proportion of the soft segment enables the polyurethane material to exhibit good mechanical properties, biocompatibility, and the like.

In the biosensor according to the first aspect of the present disclosure, optionally, the first solution comprises the first-type polymer, the first-type polymer is polyurethane, the first-type polymer has a hydrophilic group, and the proportion of the hydrophilic group in the first-type polymer is 1% to 30%. The proportion of hydrophilic groups in polyurethane affects the properties of the polyurethane material; in this case, by selecting an appropriate proportion of hydrophilic groups, the polyurethane material can exhibit good hydrophilicity and biocompatibility, etc.

In the biosensor according to the first aspect of the present disclosure, optionally, the first membrane layer has a thickness of 1 µm to 100 µm. In this case, the selection of a suitable thickness of the first membrane layer may effectively perform the intended function.

In the biosensor according to the first aspect of the present disclosure, optionally, the substance in the second membrane layer does not react with the substance in the first membrane layer. Thus, it is possible to facilitate the formation of a second membrane layer that can separate the first membrane layer from the internal structure (sensing layer and first electrode).

In the biosensor according to the first aspect of the present disclosure, optionally, the second membrane layer comprises a second-type polymer, the second-type polymer being a zwitterionic polymer or a copolymer formed from neutral monomers. In this case, the second membrane layer does not react with the two polymers in the first membrane layer, and can facilitate the formation of a second membrane layer that separates the first membrane layer from the internal structure (sensing layer and first electrode); and the second-type polymer can be dissolved using a low-corrosive or non-corrosive solvent, so that when the formed second membrane layer is in direct contact with the internal structure, the internal structure is not damaged; in addition, the second membrane layer imposes minimal restriction on analyte permeation, facilitating analyte passage through the second membrane layer to reach the sensing layer.

In the biosensor according to the first aspect of the present disclosure, optionally, the second membrane layer has a thickness of 1 µm to 30 µm. Thus, the first membrane layer can be effectively separated from the internal structure by means of the second membrane layer.

In the biosensor according to the first aspect of the present disclosure, optionally, the polymer membrane further comprises other functional membrane layers selected from any one or more of an anti-interference layer, a biocompatible layer, an antibacterial layer, a temperature compensation layer, and a signal enhancement layer. Thus, a suitable functional membrane layer can be selected as desired.

In the biosensor according to the first aspect of the present disclosure, optionally, the polymer membrane further comprises a third membrane layer, the third membrane layer being a biocompatible layer, and the first membrane layer being located between the second membrane layer and the third membrane layer. Thus, it is possible to contribute to further improving the biocompatibility of the biosensor.

In the biosensor according to the first aspect of the present disclosure, optionally, the biocompatible layer comprises a third-type polymer, which is a hydrophilic group-containing polyurethane or a cross-linked water-soluble polyurethane. In this case, the third-type polymer is also a polyurethane, which, on the one hand, may further improve the biocompatibility of the biosensor; on the other hand, it may also partially block the entry of sterilizing gas during biosensor sterilization, thereby further improving the protective effect of the polymer membrane on the biosensor.

In the biosensor according to the first aspect of the present disclosure, optionally, when the third-type polymer is the hydrophilic group-containing polyurethane, the proportion of the hydrophilic group in the third-type polymer is 20% to 50%. Thus, the biocompatible layer containing the third-type polymer can exhibit good biocompatibility, thereby further enhancing the biocompatibility of the biosensor.

In the biosensor according to the first aspect of the present disclosure, optionally, the biocompatible layer has a thickness of 1 µm to 30 µm. Thus, it is possible to contribute to improving the biocompatibility of the biosensor.

In the biosensor according to the first aspect of the present disclosure, optionally, the analyte sensed by the biosensor is one or more types.

In the biosensor according to the first aspect of the present disclosure, optionally, the sensing layer includes one or more of glucose oxidase, lactate oxidase, and hydroxybutyrate dehydrogenase. Thus, a suitable sensitive substance can be selected according to the type of analyte.

In the biosensor according to the first aspect of the present disclosure, optionally, the biosensor further comprises a second electrode different from the first electrode, and the polymer membrane also covers the second electrode. Thus, the second electrode can be protected by the polymer membrane.

A second aspect of the present disclosure provides an analyte monitoring device comprising an electronic component and a biosensor according to the first aspect of the present disclosure, the electronic component being electrically connected to the biosensor.

In accordance with the present disclosure, a biosensor and analyte monitoring device can be provided having a wider material selection range that can meet the requirements of more application scenarios.

### BRIEF DESCRIPTION OF THE DRAWINGS

FIG. 1 is a diagram showing an application scenario of the biosensor according to an example of the present disclosure.
FIG. 2 is a schematic diagram showing the biosensor according to an example of the present disclosure.
FIG. 3 is a schematic diagram showing the polymer membrane according to an example of the present disclosure.
FIG. 4 is a schematic diagram showing the polymer membrane covering an implantable portion according to an example of the present disclosure.
FIG. 5 is a topographical diagram showing the appearance of the biosensor of Example 1 of the present disclosure after sterilization.
FIG. 6 is a topographical diagram showing the appearance of the biosensor of Comparative Example 1 of the present disclosure after sterilization.

### Description of reference numerals:

100...analyte monitoring device, 1...sensor, 11...implantable portion, 12...connection portion, 13...first electrode, 14...sensing layer, 2...electronic component, 3...polymer membrane, 31...first membrane layer, 32...second membrane layer, 33...third membrane layer.

### DETAILED DESCRIPTION

Hereinafter, preferred embodiments of the present disclosure will be described in detail with reference to the accompanying drawings. In the following description, the same reference numerals are assigned to the same components, and the repeated description thereof will be omitted. In addition, the drawings are only schematic, and the proportions of the dimensions of the parts relative to each other, the shapes of the parts, etc., may be different from those in practice.

It should be noted that the terms "comprise" and "have", and any variations thereof, for example, a process, method, system, article, or apparatus that comprises or has a list of steps or elements in this disclosure are not necessarily limited to those steps or elements expressly listed, but may include or have other steps or elements not expressly listed or inherent to such process, method, article, or apparatus.

A first aspect of the present disclosure relates to a biosensor, which is a biosensor for monitoring an analyte. The biosensors of the present disclosure may be simply referred to as sensors, or may also be called monitoring probes, sensing probes, or electrode probes, among others. In some examples, the biosensors of the present disclosure are resistant to sterilizing gas and can be sterilized using gas sterilization.

In the present disclosure, the analyte may be one or more of glucose, acetylcholine, amylase, bilirubin, cholesterol, chorionic gonadotropin, creatine kinase, creatine, creatinine, DNA, fructosamine, glutamine, hormones, ketone bodies, lactic acid, lactate, oxygen, peroxide, prostate-specific antigen, prothrombin, RNA, thyroid-stimulating hormone, and troponin. This is merely illustrative; the analyte may further be other substances not shown.

In the present disclosure, the biosensor may adopt the nomenclature of "target analyte + sensor". For example, when the target analyte is glucose, the biosensor may also be referred to as a glucose sensor. As another example, when there are multiple target analytes, the biosensor may also be referred to as a multi-analyte sensor. For instance, when there are two target analytes, the biosensor may also be referred to as a dual-analyte sensor.

A second aspect of the present disclosure relates to a first membrane layer of a biosensor, the first membrane layer being capable of serving as an isolation layer. The first membrane layer can restrict the permeation of sterilizing gas during gas sterilization of the biosensor, forming an isolation barrier to reduce the impact of the sterilizing gas on the sensing layer of the biosensor (to be described later). In examples where the first membrane layer of the present disclosure functions as an isolation layer, the first membrane layer of the biosensors of the present disclosure may also be referred to as an isolation membrane, spacer layer, or sterilization layer, among others.

A third aspect of the present disclosure is directed to an analyte monitoring device, which may include an electronic component and a biosensor. The analyte monitoring devices of the present disclosure may also be referred to as continuous monitoring devices or monitoring systems, and the like. In the present disclosure, the analyte monitoring device may use the nomenclature of "target analyte of interest + monitoring device", e.g., when the target analyte of interest is glucose, the analyte monitoring device may also be referred to as a glucose monitoring device. As a further example, where there are multiple target analytes of interest, the analyte monitoring device may also be referred to as a multi-analyte monitoring device. For example, where there are two target analytes of interest, the analyte monitoring device may also be referred to as a dual analyte monitoring device.

Hereinafter, a biosensor (hereinafter may be simply referred to as a sensor) according to the present disclosure will be described with reference to the accompanying drawings, taking glucose as an example of the analyte.

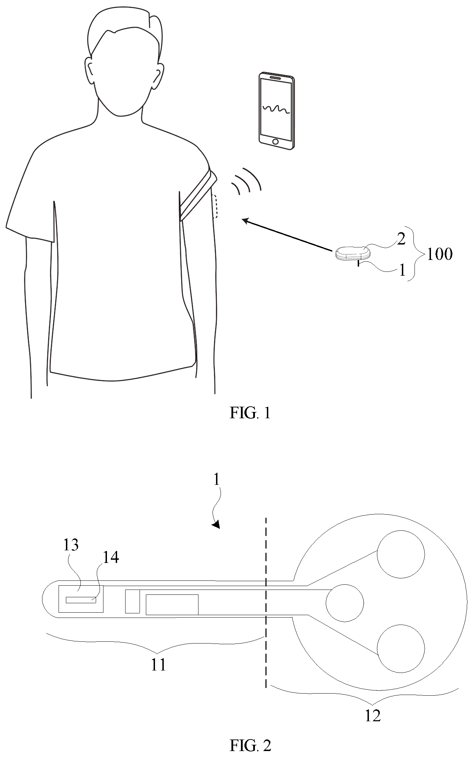

FIG. 1 is a diagram showing an application scenario of sensor 1 according to an example of the present disclosure. FIG. 2 is a schematic diagram showing sensor 1 according to an example of the present disclosure.

In some examples, the sensor 1 may be used to monitor an analyte. In some examples, the sensor 1 may be used in conjunction with an electronic component 2. In particular, referring to FIG. 1, a sensor 1 may be combined with an electronic component 2 to form an analyte monitoring device 100 such that the analyte monitoring device 100 is located on a body surface of a host, and such that the sensor 1 is located partially subcutaneously in the host, so that the analyte may be monitored by the sensor 1. It should be noted that although the application scenario of the sensor 1 is exemplarily described herein as monitoring an analyte in a host body in cooperation with the electronic component 2, the sensor 1 may also be placed in a different test environment to detect the analyte, for example, the sensor 1 may be placed in a test solution in vitro to detect the analyte; also, it is not necessarily required to combine with the electronic component 2 to form the analyte monitoring device 100 for use; for example, the sensor 1 may also be electrically connected to an external test circuit for monitoring.

In some examples, the sensor 1 may include an implantable portion 11 (see FIG. 2). The implantable portion 11 may be implanted subcutaneously in the host, and in use, the implantable portion 11 may be located subcutaneously in the host in contact with the analyte. Thus, the analyte in the host can be monitored by the sensor 1. In some examples, the sensor 1 may include a connection portion 12 (see FIG. 2). The implantable portion 11 may be electrically connected to the electronic component 2 via the connection portion 12. In use, the implantable portion 11 may be located subcutaneously, and the connection portion 12 may be located extracorporeally, therefore, the connection portion 12 may also be referred to as an extracorporeal portion.

In some examples, the sensor 1 may include a first electrode 13 (see FIG. 2). A sensing layer 14 may be provided on the first electrode 13. The analyte can react upon contact with the sensing layer 14 on the first electrode 13 to produce an electrical signal, and analytical processing of this signal can yield information about the analyte level. Accordingly, the first electrode 13 may also be referred to as a working electrode.

In some examples, the sensor 1 may include a second electrode. Here, the second electrode may be the same as or different from the first electrode 13. For example, the second electrode may be a reference electrode or a counter electrode. As another example, the second electrode may be a second working electrode, and the analyte sensed by the second working electrode may be the same as or different from that sensed by the first electrode 13. In some examples, the sensor 1 may include a third electrode. The third electrode may be a reference electrode or a counter electrode and may be different from the second electrode. In some examples, the sensor 1 may include a working electrode, a reference electrode, and a counter electrode. Thus, a sensor 1 having a three-electrode system can be formed to monitor an analyte. In some examples, the sensor 1 may include a fourth electrode. The fourth electrode may be a second working electrode, a reference electrode, a counter electrode, or a blank electrode for improving the measurement accuracy of the sensor 1. In some examples, the fourth electrode may be different from the first electrode, the second electrode, and the third electrode.

In some examples, the sensed analyte of sensor 1 may be one or more. In some examples, the analyte may be selected from any one or more of glucose, lactate, and ketone bodies. Where the analyte is a ketone body, the level of the ketone body can be reflected by monitoring the level of beta-hydroxybutyrate.

In some examples, sensing layer 14 may include a sensitive substance that may promote an analyte reaction. The sensing material may be selected in accordance with the subject. For example, when the analyte is glucose, the sensing layer 14 may include glucose oxidase. In some examples, sensing layer 14 may include one or more of glucose oxidase, lactate oxidase, and hydroxybutyrate dehydrogenase. Thus, a suitable sensitive substance can be selected according to the type of analyte. In some examples, the glucose enzyme may be glucose oxidase or glucose dehydrogenase. In some examples, the sensing layer 14 may be formed by applying an enzyme solution containing a sensing substance to the working electrode. In some examples, sensing layer 14 may be formed by spin coating, dip-coating, drop casting, spray coating, printing, or the like.

In some examples, when the analytes are plural, a sensing layer corresponding to each analyte may be provided on the first electrode, respectively, or a plurality of working electrodes may be provided, and a sensing layer corresponding to each analyte may be provided on each working electrode. For example, in some examples, sensor 1 may be a dual-analyte sensor that can simultaneously monitor glucose and ketone. Two working electrodes may be provided, and a glucose-sensing layer including glucose oxidase and a ketone-sensing layer including hydroxybutyrate dehydrogenase may be provided on the two working electrodes, respectively.

In some examples, the sensor 1 may include a polymer membrane 3 (illustrated later).

In some examples, the polymer membrane 3 may control the diffusion of the analyte. That is, the polymer membrane 3 may have a permselectivity/diffusion-limiting effect on the analyte. In some examples, the polymer membrane 3 may cover at least the sensing layer 14. Thus, the amount of permeation of the analyte, i.e., the amount of analyte entering the sensing layer 14, can be controlled by the polymer membrane 3, thereby helping to maintain the magnitude of the electrical signal generated by the analyte in contact with the sensing layer 14 within the linear response range of the sensor 1.

In some examples, the polymer membrane 3 may have biocompatibility. In some examples, the polymer membrane 3 may cover the implantable portion 11 of the sensor 1 (illustrated later). Thus, sensor 1 may exhibit biocompatibility. In the present disclosure, "biocompatible" means that the cell relative survival rate in the cytotoxicity test result is more than 90%, indicating good biocompatibility.

In some examples, the polymer membrane 3 may include a functional membrane layer. The functional membrane layer may be provided based on the application requirements. For example, the functional membrane layer may be selected from any one or more of a sterilization layer, an anti-interference layer, a biocompatible layer, an antibacterial layer, a temperature-compensation layer, and a signal-enhancement layer. Thus, it is possible to select a suitable functional membrane layer as required to adapt the sensor 1 to more application scenarios. In some examples, the functional membrane layer may also be other known functional membrane layers, not limited to the ones described in this disclosure.

In some examples, the polymer membrane 3 may limit the permeation of the sterilizing gas when the sensor 1 is subjected to gas sterilization. For example, the polymer membrane 3 may limit the permeation of ethylene oxide (which may be simply referred to as EO). Thus, a sensor 1 resistant to the sterilizing gas can be provided. Accordingly, the sensor 1 of the present disclosure can be sterilized using gas sterilization, and compared to a sensor 1 without the polymer membrane 3, the decrease in sensitivity of the sensor 1 before and after sterilization is greatly reduced, and high sensitivity can be maintained after sterilization. In some examples, the polymer membrane 3 may cover at least the sensing layer 14 and the first electrode 13. Thus, corrosion of the internal structure (sensing layer 14 and first electrode 13) by the sterilizing gas during sterilization can be reduced, thereby protecting the internal structure.

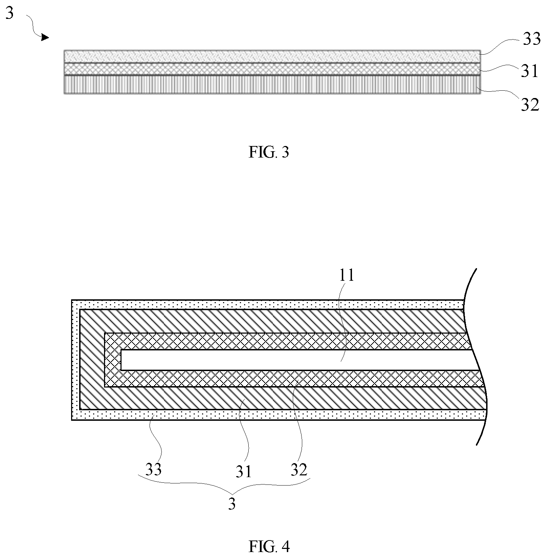

FIG. 3 is a schematic diagram illustrating a polymer membrane 3 according to an example of the present disclosure.

In some examples, the polymer membrane 3 may include a first membrane layer 31 (see FIG. 3). In some examples, the polymer membrane 3 may include a second membrane layer 32 (see FIG. 3). In some examples, the second membrane layer 32 may be closer to the first electrode 13 than the first membrane layer 31.

In some examples, the first membrane layer 31 may include a substance that would damage the sensing layer 14 and/or the first electrode 13 upon direct contact. In some examples, the second membrane layer 32 may separate the first membrane layer 31 from the sensing layer 14 and the first electrode 13. In this case, the first membrane layer 31 includes substances harmful to the sensing layer 14 and/or the first electrode 13; by providing the second membrane layer 32, the first membrane layer 31 is spaced from the internal structure (sensing layer 14 and first electrode 13), thereby reducing adverse effects caused by these harmful substances in the first membrane layer 31 (such as corrosion-induced structure integrity degradation and functional loss) on the sensing layer 14 and the first electrode 13.

Accordingly, in the present disclosure, the first membrane layer 31 containing substances that negatively affect the electrode structure is innovatively selected, and the design of the second membrane layer 32 overcomes the performance bottleneck caused by limited material choices in conventional designs, which expands the material selection range for the functional membrane layer (first membrane layer 31), enhances the performance and multifunctionality of sensor 1, improves the stability and reliability of sensor 1, and provides new ideas and possibilities for innovation and application expansion in biosensor technology. Thus, a sensor 1 with an expanded material selection range can be provided, meeting requirements across a broader range of application scenarios.

In the present disclosure, the second membrane layer 32, which separates the first membrane layer 31 from the internal structure, may also be referred to as a protective layer.

In some examples, the substance in the second membrane layer 32 may not react with the substance in the first membrane layer 31. Thus, this facilitates the formation of a second membrane layer 32 that can separate the first membrane layer 31 from the internal structures (sensing layer 14 and first electrode 13).

In some examples, the first membrane layer 31 may be fabricated from a first solution. The first solution may be applied to a target location to form the first membrane layer 31. Here, the target location refers to where formation of the first membrane layer 31 is desired. For example, the first solution coating may be applied onto the implantable portion 11 to form the first membrane layer 31. In some examples, the first membrane layer 31 may be formed by applying the first solution to the target location via spin coating, dip-coating, drop casting, spray coating, or other methods.

In some examples, the first solution may comprise a first-type polymer and a solvent. The specific type of the first-type polymer may be selected based on the desired functionality. For example, in cases where the first membrane layer 31 requires biocompatibility, the first-typepolymer may be chosen as a biocompatible material. As another example, when the first membrane layer 31 serves as an anti-interference layer, the first-typepolymer may be selected to exhibit resistance against specific interferents.

In some examples, the solvent in the first solution may be selected as a substance capable of dissolving the first-type polymer. In some examples, the solvent in the first solution may cause damage to the sensing layer 14 and/or the first electrode 13 upon direct contact. In this case, selecting a solvent with stronger solvating power capable of dissolving certain specific polymers (e.g., some non-polar or highly polar solvents can dissolve more types of polymers, including those difficult to process with conventional solvents) helps broaden the material selection range for polymers in the first membrane layer 31.

In some examples, the first membrane layer 31 may be configured to reduce the effect of the sterilizing gas on the sensing layer 14 when sensor 1 is subjected to gas sterilization. In this case, the sensor 1 of the present disclosure can be sterilized using a gas sterilization method, and compared to a biosensor without the first membrane layer 31, the decrease in sensitivity before and after sterilization is significantly reduced, and high sensitivity may be maintained after sterilization. Thus, a sensor 1 resistant to the sterilizing gas can be provided. In the present disclosure, the first membrane layer 31, which is configured to reduce the effect of the sterilizing gas on the sensing layer 14 during gas sterilization of sensor 1, may also be referred to as an isolation layer or a sterilization layer. In some examples, ethylene oxide (EO) may be used to sterilize sensor 1 of the present disclosure.

The polymer membrane 3 of the present disclosure will now be described with reference to an example in which the sterilizing gas is ethylene oxide (EO) and the first membrane layer 31 is configured to reduce the effect of the sterilizing gas on the sensing layer 14 during gas sterilization of sensor 1.

In some examples, the first membrane layer 31 may comprise a first-type polymer. In other words, in some examples, the first solution may comprise a first-type polymer. In some examples, the first-type polymer may limit the permeation of sterilizing gas (e.g., ethylene oxide (EO)). Thus, the first membrane layer 31 formed from a first solution comprising the first-type polymer is capable of limiting the permeation of sterilizing gas.

In some examples, the first membrane layer may have a diffusion-limiting effect on the analyte sensed by the sensor 1. In some examples, the first membrane layer may have biocompatibility.

In some examples, the first-type polymer may be polyurethane. Polyurethane is a material with good physical and mechanical properties, blood compatibility, and biocompatibility, and ethylene oxide at room temperature and atmospheric pressure typically does not cause structural changes or degradation of polyurethane. In this case, by providing the first membrane layer 31 comprising polyurethane, the permeation of sterilizing gas during gas sterilization of sensor 1 can be limited, forming an isolation barrier that reduces the impact of sterilizing gas on the sensing layer 14, particularly the effect of gaseous ethylene oxide on active substances (e.g., sensitive substances) in the sensing layer 14; furthermore, the first membrane layer 31 comprising polyurethane exhibits good biocompatibility, improving the safety of sensor 1 when implanted subcutaneously in a host; in addition, the first membrane layer 31 has a permeation-limiting effect on specific analytes (e.g., glucose), enabling the polymer membrane 3 to function as a semi-permeable membrane. In some examples, the first-type polymer may have urethane groups.

In some examples, the first-type polymer may have soft segments, and the proportion of soft segments in the first-type polymer may range from 5% to 50%. In this case, the properties of the first-type polymer can be tuned by adjusting the proportion of soft segments. In some examples, preferably, the proportion of soft segments is from 10% to 25%. In some examples, when the first-type polymer is polyurethane, the polyurethane may have soft segments. Since the proportion of the soft segment affects the properties of the polyurethane material, in this case, selecting an appropriate proportion of the soft segment enables the polyurethane material to exhibit good mechanical properties, biocompatibility, and the like. In some examples, the permeability of the first membrane layer 31 to the analyte and the biocompatibility of the first membrane layer 31 itself can be adjusted by modifying the proportion of soft segments in the polyurethane, thereby tuning properties such as elasticity, modulus, flexibility, and water absorption. In some examples, the soft segment may include an epoxy-functional group-containing component. For example, the soft segment may comprise polyethylene oxide (PEO).

In some examples, the polyurethane may be a block copolymer composed of soft segments and hard segments. In some examples, in polyurethanes, the soft segments may be composed of hydroxyl-containing molecules such as polyester polyols, polyether polyols, polycaprolactones, polylactic-co-glycolic acid copolymers, and the like, of varying molecular weights. In some examples, in polyurethane, the hard segments may be composed of compounds such as diisocyanates and low molecular weight short-chain extenders. In some examples, the diisocyanate may be an aliphatic isocyanate. For example, the diisocyanate may be 1,6-hexamethylene diisocyanate (HDI), 4,4'-dicyclohexylmethane diisocyanate (HMDI), or isophorone diisocyanate (IPDI). In some examples, the short-chain extender may be 1,4-butanediol.

In some examples, the polyurethane may be polycarbonate polyurethane, polyether-based polyurethane, and/or polyester-based polyurethane. In some examples, the polyurethane may include one or more of aliphatic polyether-based polyurethane, aliphatic polyether-based polyurethane urea, cycloaliphatic polyether-based polyurethane, cycloaliphatic polyether-based polyurethane urea, aromatic polyether-based polyurethane, aromatic polyether-based polyurethane urea, aliphatic polyester-based polyurethane, aliphatic polyester-based polyurethane urea, cycloaliphatic polyester-based polyurethane, cycloaliphatic polyester-based polyurethane urea, aromatic polyester-based polyurethane, and aromatic polyester-based polyurethane urea.

In some examples, the first-type polymer may be a zwitterionic polyurethane. In this case, this type of polyurethane has the flexibility and extensibility of polyether chains while possessing the characteristics of zwitterionic groups, and zwitterionic groups with different properties can be introduced as needed to adjust the properties of the first membrane layer 31. In some examples, the zwitterionic groups in the zwitterionic polyurethane may include amine groups, carboxyl groups, and/or sulfonic acid groups. In this case, the hydrophilicity and biocompatibility of the first membrane layer 31 may be improved by introducing specific zwitterionic groups.

In some examples, the first-type polymer may have hydrophilic groups. Thus, the first-type polymer may exhibit a certain degree of hydrophilicity, which is beneficial for improving the biocompatibility of the first membrane layer 31; and ethylene oxide (EO) can react with certain types of hydrophilic groups; Accordingly, the first-type polymer containing hydrophilic groups capable of reacting with ethylene oxide can adsorb ethylene oxide to some extent, thereby further enhancing the barrier effect of the first membrane layer 31 against ethylene oxide. In some examples, the hydrophilic groups may include hydroxyl groups, carboxyl groups, and/or ether groups.

In some examples, the proportion of hydrophilic groups in the first-type polymer may be from 1% to 30%. For example, in the first-type polymer, the proportion of hydrophilic groups may be 1%, 2%, 5%, 6%, 8%, 10%, 12%, 13%, 15%, 16%, 17%, 18%, 20%, 22%, 25%, 26%, 27%, 28%, 29%, or 30%. The proportion of hydrophilic groups in the first-type polymer affects its performance; in this case, the properties of the first-type polymer may be adjusted by varying the proportion of hydrophilic groups, thereby tuning the performance of the first membrane layer 31.

In some examples, when the first-type polymer is a polyurethane, the proportion of hydrophilic groups may be from 1% to 30%. In this case, by selecting a suitable proportion of hydrophilic groups, the polyurethane material can have good hydrophilicity and biocompatibility, etc.; furthermore, polyurethane containing hydrophilic groups of a type capable of reacting with ethylene oxide can absorb part of the ethylene oxide during sterilization and introduce epoxy groups, thereby further improving the barrier effect of the first membrane layer 31 against sterilizing gas. In some examples, preferably, the proportion of hydrophilic groups in the first-type polymer may be from 10% to 25%. In some examples, the permeability coefficient of the first membrane layer 31 to the analyte may be adjusted by modifying the hydrophilic groups in the polyurethane. For example, increasing the proportion of hydrophilic groups in the polyurethane can enhance its dispersibility in the liquid phase, thereby contributing to an increased permeability of the first membrane layer 31 to the analyte. Conversely, the permeability of the first membrane layer 31 to the analyte may be reduced by reducing the proportion of hydrophilic groups in the polyurethane. In some examples, the hydrophilicity of the polyurethane may be increased by introducing a block containing a hydrophilic group.

In some examples, the first solution may include an organic solvent. The first-type polymer may be dissolved in an organic solvent. Thus, the formation of the first solution and, subsequently, the formation of the first membrane layer 31 can be facilitated. In some examples, the organic solvent may be a mixed solvent of any one or more of tetrahydrofuran, dichloromethane, and N,N-dimethylacetamide. Thus, the first-type polymer may be dissolved using an organic solvent.

In some examples, the mass fraction of the first-type polymer in the first solution may range from 0.1% to 15%. For example, the mass fraction of the first-type polymer in the first solution may be 0.1%, 0.5%, 1%, 2%, 3%, 4%, 5%, 6%, 7%, 8%, 9%, 10%, 11%, 12%, 13%, 14%, or 15%. As a result, this mass fraction can help the first membrane layer 31 limit the permeation of the sterilizing gas during gas sterilization. In some examples, preferably, the mass fraction of the first-type polymer in the first solution may range from 0.1% to 5%. In this case, the first membrane layer 31 is capable of limiting the permeation of the sterilizing gas while also providing a suitable analyte permeability coefficient.

In some examples, the first membrane layer 31 may have a thickness of 1 µm to 100 µm. In this case, selecting an appropriate thickness for the first membrane layer 31 enables it to effectively perform its intended function. It should be noted that different functional layers may have different thicknesses according to actual needs. For example, in some examples where the first membrane layer 31 is an isolation layer, preferably, the first membrane layer 31 may have a thickness of 1 µm to 40 µm. For example, the first membrane layer 31 may have a thickness of 1 µm, 5 µm, 8 µm, 10 µm, 12 µm, 15 µm, 18 µm, 20 µm, 22 µm, 25 µm, 28 µm, 30 µm, 32 µm, 35 µm, 38 µm, or 40 µm. In this case, selecting an appropriate thickness for the first membrane layer 31 enables it to effectively function as an isolation barrier, thereby improving the resistance of sensor 1 to the sterilizing gas; in addition, it may also allow the first membrane layer 31 to provide a suitable analyte permeability coefficient. In some examples, preferably, the first membrane layer 31 may have a thickness of 10 µm to 30 µm. In this case, it may help balance the shielding effect of the first membrane layer 31 against the sterilizing gas and the analyte permeability coefficient.

In some examples, as previously described, the polymer membrane 3 may include a second membrane layer 32. In some examples, the second membrane layer 32 may be used to separate the sensing layer 14 from the first membrane layer 31. That is, the second membrane layer 32 may be closer to the first electrode 13 than the first membrane layer 31. As a result, the adverse effects of the organic solvent in the first membrane layer 31 on the sensing layer 14 and the first electrode 13 (e.g., corrosion resulting in impaired internal structure integrity and functional loss) can be mitigated.

In some examples, the second membrane layer 32 may be made from a second solution. In some examples, the second solution may be applied to the implantable portion 11 to form the second membrane layer 32. In some examples, the second membrane layer 32 may be formed by applying the second solution to the target position via spray coating, spin coating, dip coating, or the like.

In some examples, the second solution may include a second-type polymer. In other words, the second membrane layer 32 may include a second-type polymer. The second-type polymer may be non-reactive with the first-type polymer. As a result, it can help form a second membrane layer 32 that can separate the first membrane layer 31 from the internal structures (the sensing layer 14 and the first electrode 13).

In some examples, the second-type polymer may be a zwitterionic polymer or a copolymer formed from neutral monomers. In this case, the second membrane layer 32 does not react with the two polymers in the first membrane layer 31; and a low-corrosive or non-corrosive solvent can be used to dissolve the second-type polymer, so that the formed second membrane layer 32 does not damage the internal structure upon direct contact with the internal structure (the sensing layer 14 and the first electrode 13); and the formed second membrane layer 32 imposes minimal restriction on analyte permeation, thereby allowing the analyte to pass through the second membrane layer 32 and contact the sensing layer 14; in addition, the second-type polymer is also insoluble in the organic solvent of the first solvent, thereby reducing the possibility of delamination of the formed membrane and suppressing interaction between the two membrane layers, and improving the overall stability of the polymer membrane 3.

In some examples, the second-type polymer may be chitosan or carboxymethacrylate. In some examples, the second-type polymer may be selected from one or more of the following: polyethylene glycol-block-polystyrene, polyacrylic acid-block-polystyrene, polyacrylic acid-co-polystyrene, polyacrylamide-block-polystyrene, polyacrylamide-co-polystyrene, poly(2-vinylpyridine)-block-polystyrene, poly(4-vinylpyridine)-co-polystyrene, poly(4-vinylpyridine)-copolyvinylpyrrolidone, poly(2-vinylpyridine)-co-polystyrene, poly(4-vinylpyridine)-block-polystyrene, poly(4-vinylpyridine)-co-polyacrylamide, poly(ethoxyethyl acrylate)-co-poly(hydroxyethyl acrylate), and poly(ethoxypropyl acrylate)-co-poly(vinyl alcohol).

In some examples, the solvent of the second solution may be a lower alcohol solution, an inorganic salt buffer solution, or a mixed solution of a lower alcohol and an inorganic salt buffer solution. Thus, the second polymer can be dissolved, facilitating the subsequent application of the second solution. In some examples, the solvent of the second solution may be methanol, ethanol, or a mixed solution of ethanol and a phosphate buffer solution.

In some examples, the second membrane layer 32 may have a thickness of 1 µm to 30 µm. For example, the second membrane layer 32 may have a thickness of 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 22 µm, 22 µm, 23 µm, 24 µm, 25 µm, 26 µm, 27 µm, 28 µm, 29 µm, or 30 µm. Thus, the second membrane layer 32 can effectively separate the first membrane layer 31 from the internal structure. In some examples, preferably, the second membrane layer 32 may have a thickness of 1 µm to 20 µm. In some examples, preferably, the second membrane layer 32 may have a thickness of 8 µm to 15 µm. In some examples, preferably, the second membrane layer 32 may have a thickness of no less than 10 µm.

In some examples, the thickness ratio of the second membrane layer 32 to the first membrane layer 31 may be from 1:1 to 1:10. For example, the thickness ratio of the second membrane layer 32 to the first membrane layer 31 may be 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, or 1:10. As a result, the second membrane layer 32 can help separate the first membrane layer 31 from the internal structure (the sensing layer 14 and the first electrode 13). In some examples, preferably, the thickness ratio of the second membrane layer 32 to the first membrane layer 31 may be from 1:1 to 1:5.

In some examples, the polymer membrane 3 may further include other functional membrane layers. Here, the "other functional membrane layer" refers to any membrane layer other than the first membrane layer 31 and the second membrane layer 32. By incorporating other functional membrane layers, the polymer membrane 3 can achieve additional desired functionalities. For example, in some examples, the other functional membrane layer may be selected from one or more of an anti-interference layer, a biocompatible layer, an antibacterial layer, a temperature-compensation layer, and a signal-enhancement layer. Thus, a suitable functional membrane layer may be selected as desired. In some examples, the other functional membrane layers may also be other known functional membrane layers, and are not limited to those described in this disclosure.

For example, in some examples, the polymer membrane 3 may include a third membrane layer 33 (see FIG. 3). The third membrane layer 33 may have biocompatibility and serve as the outermost layer of the polymer membrane 3 (i.e., the membrane layer furthest from the first electrode 13). In this case, it can be advantageous to improve the biocompatibility of the sensor 1. That is, in some examples, the third membrane layer 33 may be a biocompatible layer.

In some examples, the first membrane layer 31 may be located between the third membrane layer 33 and the second membrane layer 32. It should be noted that, in the present disclosure, the first membrane layer 31 can already have good biocompatibility by adjusting the first membrane layer 31, and the additional third membrane layer 33 is added for the purpose of further improving the biocompatibility of the sensor 1 and improving reliability, and the third membrane layer 33 does not necessarily need to be provided.

In some examples, the third membrane layer 33 may be made from a third solution. In some examples, the third solution may be applied to the implantable portion 11 to form the third membrane layer 33. In some examples, the third membrane layer 33 may be formed by applying the third solution to the target position via spray coating, spin coating, dip coating, or the like. In some examples, the third solution may include a third-type polymer. In other words, the third membrane layer 33 may include a third-type polymer. The third-type polymer may be a polymer having biocompatibility. Thus, the third membrane layer 33 having biocompatibility can be formed, thereby helping improve biocompatibility of the sensor 1.

In some examples, the third-type polymer may be polyurethane. In this case, the third-type polymer is also polyurethane, which, on the one hand, may further improve the biocompatibility of the sensor 1; on the other hand, it may also partially block the entry of sterilizing gas during the sterilization process of the sensor 1, further improving the protective effect of the polymer membrane 3 on the sensor 1.

In some examples, the third-type polymer may be a hydrophilic-group-containing polyurethane. In this case, the third membrane layer 33 comprising the third-type polymer can have good biocompatibility; in addition, polyurethane containing hydrophilic groups capable of reacting with ethylene oxide can react with ethylene oxide during sterilization, absorb part of the ethylene oxide, and introduce epoxy groups, further improving the hydrophilicity of the third membrane layer 33, thereby helping improve the biocompatibility of the polymer membrane 3.

In some examples, the proportion of hydrophilic groups in the third-type polymer may be from 20% to 50%. For example, in the third-type polymer, the proportion of hydrophilic groups may be 20%, 22%, 25%, 28%, 30%, 32%, 35%, 37%, 40%, 42%, 45%, 48%, or 50%. Thus, the third membrane layer 33 including the third-type polymer, can have good biocompatibility, thus further improving the biocompatibility of the sensor 1. In some examples, preferably, in the third-type polymer, the proportion of hydrophilic groups may be from 30% to 50%. In some examples, preferably, in the third-type polymer, the proportion of hydrophilic groups may be not less than 30%.

In some examples, the third-type polymer may be a cross-linked water-soluble polyurethane. In this case, the water-soluble polyurethane has good biocompatibility, as a result the third membrane layer 33 can be formed with good biocompatibility. In some examples, the third-type polymer may be polyethylene glycol block polyurethane, polymethacrylate block polyurethane, polyvinyl alcohol block polyurethane, or polypropylene alcohol block polyurethane.

In some examples, the solvent of the third solution may be an organic solvent or a mixed solvent. The third-type polymer may be dissolved in an organic solvent. As a result, the formation of the third solution can be facilitated, thereby further enabling the formation of the third membrane layer 33. In some examples, the organic solvent may be a mixed solvent of any one or more of tetrahydrofuran, dichloromethane, and N,N-dimethylacetamide. Thus, the third-type polymer can be dissolved using an organic solvent. In some examples, the solvent of the third solution may include water. Thus, dissolution of the third-type polymer can be facilitated.

In some examples, the mass fraction of the third-type polymer in the third solution may be from 0.1% to 15%. For example, the mass fraction of the third-type polymer in the third solution may be 0.1%, 0.5%, 1%, 2%, 3%, 4%, 5%, 6%, 7%, 8%, 9%, 10%, 11%, 12%, 13%, 14%, or 15%. Thus, the third membrane layer 33 with good biocompatibility can be formed. In some examples, preferably, the mass fraction of the first-type polymer in the first solution may be from 0.1% to 5%.

In some examples, the third membrane layer 33 may have a thickness of 1 µm to 30 µm. For example, the third membrane layer 33 may have a thickness of 1 µm, 3 µm, 5 µm, 6 µm, 8 µm, 10 µm, 12 µm, 14 µm, 15 µm, 16 µm, 18 µm, 20 µm, 22 µm, 25 µm, 26 µm, 28 µm, or 30 µm. Thus, the biocompatibility of the sensor 1 can be improved. In some examples, preferably, the third membrane layer 33 may have a thickness of 10 µm to 20 µm.

FIG. 4 is a schematic diagram showing the polymer membrane 3 covering the implantable portion 11 according to an example of the present disclosure. In FIG. 4, the scale of the polymer membrane 3 is exaggerated for clarity, and the structure of the implantable portion 11 is simplified, but this should not be construed as limiting the present disclosure.

In some examples, the polymer membrane 3 may cover the implantable portion 11 (see FIG. 4). In some examples, the polymer membrane 3 may cover the electrodes on the implantable portion 11. For example, the polymer membrane 3 may cover the first electrode 13 and the second electrode. Thus, the first electrode 13 and the second electrode can be protected by the polymer membrane 3. For another example, the polymer membrane 3 may cover the first electrode 13, the second electrode, and the third electrode on the implantable portion 11. Thus, the electrodes can be protected by the polymer membrane 3. In some examples, the polymer membrane 3 may completely cover the implantable portion 11. Thus, the safety of the sensor 1 when implanted subcutaneously in a host can be improved.

In some examples, the polymer membrane 3 may have a thickness of no greater than 100 µm. In this case, stable adhesion of the polymer membrane 3 to the implantable portion 11 can be facilitated, and the overall space occupied by the implantable portion 11 is small, which may reduce discomfort after implantation subcutaneously in a host and thereby improve wearing comfort.

In some examples, the polymer membrane 3 may have a thickness of 15 µm to 200 µm. In some examples, the polymer membrane 3 may have a thickness of 15 µm to 100 µm. For example, the polymer membrane 3 may have a thickness of 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, or 100 µm. In some examples, preferably, the polymer membrane 3 may have a thickness of 30 µm to 60 µm.

In some examples, the sensor 1 may include a substrate. In some examples, the various electrodes of the sensor 1 may be disposed on the substrate. For example, the first electrode, the second electrode, the third electrode, and/or the fourth electrode may be disposed on the substrate. In some examples, the substrate may be a flexible substrate. The flexible substrate may be made of at least one of polyethylene (PE), polypropylene (PP), polyimide (PI), polystyrene (PS), polyethylene terephthalate (PET), and polyethylene naphthalate (PEN). In addition, in other examples, the flexible substrate may also be made substantially of a metal foil, ultra-thin glass, a single-layer inorganic membrane, a multilayer organic thin membrane, or a multilayer inorganic thin membrane. In some examples, the substrate may also be a rigid substrate. The rigid substrate may generally comprise a ceramic having weak electrical conductivity, alumina, silica, or the like. Thus, it can be implanted into a body surface (e.g., a superficial layer of skin, etc.) without the need for an auxiliary implantation device (e.g., an insertion needle).

In some examples, the electrodes of the sensor 1 may each independently be made of at least one of gold, glassy carbon, graphite, platinum, silver, silver chloride, palladium, titanium, or iridium. Thus, it is possible to have good electrical conductivity without affecting the electrochemical reactions of the respective electrodes.

In some examples, preferably, the sensor 1 may comprise a first electrode 13 provided with a sensing layer 14, and a polymer membrane 3, the polymer membrane 3 may cover at least the sensing layer 14 and the first electrode 13, the polymer membrane may comprise a first membrane layer 31 and a second membrane layer 32, the second membrane layer 32 may be closer to the first electrode 13 than the first membrane layer 31, the first membrane layer 31 may be configured to reduce the influence of the sterilizing gas on the sensing layer 14 when the sensor 1 is subjected to gas sterilization, the first membrane layer 31 may be made from a first solution, the first solution may include a first-type polymer and an organic solvent, and the first-type polymer may be polyurethane. In this case, the polymer membrane 3 of the sensor 1 comprises a second membrane layer 32 and a first membrane layer 31. By providing the first membrane layer 31 comprising polyurethane, the permeation of the sterilizing gas can be limited during gas sterilization of the sensor 1, forming an isolation barrier to reduce the impact of the sterilizing gas on the sensing layer 14, particularly the effect of gaseous ethylene oxide on the active substance in the sensing layer 14, enabling the sensor 1 to be sterilized using a gas sterilization method. Compared with a biosensor without the first membrane layer 31, the biosensor disclosed herein exhibits a significantly reduced decrease in sensitivity before and after sterilization and can still maintain high sensitivity after sterilization. Furthermore, the first membrane layer 31 comprising polyurethane has good biocompatibility, which can improve the safety of the sensor 1 when located subcutaneously in a host; in addition, the first membrane layer 31 also has a permeation-limiting effect on the analyte, and can function as a semi-permeable membrane; in addition, the provision of the second membrane layer 32 can separate the first membrane layer 31 from the internal structure (the sensing layer 14 and the first electrode 13), mitigating the adverse effects of organic solvents in the first membrane layer 31 on the sensing layer 14 and the first electrode 13 (e.g., corrosion resulting in compromised structure integrity and functional loss). Thus, a sensor 1 resistant to the sterilizing gas can be provided.

In some examples, preferably, the sensor 1 comprises a first electrode 13 provided with a sensing layer 14, and a polymer membrane 3, the polymer membrane 3 covering at least the sensing layer 14 and the first electrode 13, the polymer membrane comprising a first membrane layer 31 and a second membrane layer 32, the first membrane layer 31 comprising a substance that causes damage to the sensing layer 14 and the first electrode 13 upon direct contact, the second membrane layer 32 being closer to the first electrode 13 than the first membrane layer 31 to separate the first membrane layer 31 from the sensing layer 14 and the first electrode 13.

Embodiments of the present disclosure may include:
A. A biosensor comprising a first electrode provided with a sensing layer, and a polymer membrane covering at least the sensing layer and the first electrode, the polymer membrane comprising a first membrane layer and a second membrane layer, the first membrane layer comprising a substance that causes damage to the sensing layer and/or the first electrode upon direct contact, the second membrane layer being closer to the first electrode than the first membrane layer to separate the first membrane layer from the sensing layer and the first electrode.
B. A biosensor, the sensor comprising a first electrode provided with a sensing layer, and a polymer membrane, the polymer membrane covering at least the sensing layer and the first electrode, the polymer membrane comprising a first membrane layer and a second membrane layer, the second membrane layer being closer to the first electrode than the first membrane layer, the first membrane layer being configured to reduce the effect of a sterilizing gas on the sensing layer when the sensor is subjected to gas sterilization.

In some examples, embodiment A and embodiment B can each be independently combined with any one or more of the following additional elements. Element 1: the first membrane layer includes a substance that can cause damage to the sensing layer and/or the first electrode upon direct contact. Element 2: the first membrane layer may be configured to reduce the effect of the sterilizing gas on the sensing layer during gas sterilization of the sensor.

Element 3: the first membrane layer is made from a first solution comprising a first-type polymer and a solvent. Element 4: the first-type polymer is polyurethane. Element 5: the polyurethane may be polycarbonate polyurethane, polyether-based polyurethane, and/or polyester-based polyurethane. Element 6: the mass fraction of the first-type polymer is from 0.1% to 15%. Element 7: the solvent of the first solution may cause damage to the sensing layer and/or the first electrode upon direct contact. Element 8: the solvent of the first solution is an organic solvent. Element 9: the first-type polymer may be a zwitterionic polyurethane. Element 10: the first-type polymer has a soft segment. Element 11: in the first-type polymer, the proportion of soft segments is from 5% to 50%. Element 12: the first-type polymer has hydrophilic groups. Element 13: in the first-type polymer, the proportion of hydrophilic groups is from 1% to 30%. Element 14: the first membrane layer has a thickness of 1 µm to 100 µm.

Element 15: the second membrane layer may include a second-type polymer. Element 16: the material in the second membrane layer may be non-reactive with the material in the first membrane layer. Element 17: the second-type polymer may be non-reactive with the first-type polymer. Element 18: the second-type polymer may be a zwitterionic polymer or a copolymer formed from neutral monomers. Element 19: the second membrane layer may have a thickness of 1 µm to 30 µm. Element 20: the thickness ratio of the second membrane layer to the first membrane layer may be from 1:1 to 1:10.

Element 21: the polymer membrane may further include other functional membrane layers selected from any one or more of an anti-interference layer, a biocompatible layer, an antibacterial layer, a temperature-compensation layer, and a signal-enhancement layer. Element 22: the polymer membrane further includes a third membrane layer, the third membrane layer being a biocompatible layer, the first membrane layer being located between the second membrane layer and the third membrane layer. Element 23: the biocompatible layer includes a third-type polymer, which is a hydrophilic group-containing polyurethane or a cross-linked water-soluble polyurethane.

Element 24: the analyte sensed by the biosensor is one or more analytes. Element 25: the sensing layer includes one or more of glucose oxidase, lactate oxidase, and hydroxybutyrate dehydrogenase. Element 26: when the analytes are multiple, sensing layers corresponding to respective analytes may be provided on the first electrode, or multiple working electrodes may be provided with sensing layers corresponding to respective analytes disposed on each working electrode. Element 27: the sensor may be a dual-analyte sensor capable of simultaneously monitoring glucose and ketone. Element 28: the first membrane layer may have a permselectivity/diffusion-limiting effect on the analyte. Element 29: the first membrane layer may have biocompatibility.

In summary, in a first aspect of the present disclosure, a sensor 1 having an expanded range of material selection range can be provided. In particular, a sensor 1 resistant to the sterilizing gas can also be provided.

As previously described, a second aspect of the present disclosure provides a first membrane layer 31 of the sensor 1. The first membrane layer 31 may limit the permeation of the sterilizing gas during gas sterilization of the sensor 1, forming an isolation barrier to reduce the influence of the sterilizing gas on the sensing layer 14 of the sensor 1. That is, the second aspect of the present disclosure provides an isolation layer of the sensor 1.

The first membrane layer 31 according to the second aspect of the present disclosure is consistent with the first membrane layer 31 in the sensor 1 described in the first aspect of the present disclosure, and specific components and proportions may be referred to in the description of the first aspect, and will not be repeated herein.

In some examples, the first membrane layer 31 may be configured to reduce the effect of the sterilizing gas on the sensing layer 14 when the sensor 1 is subjected to gas sterilization. In some examples, the first membrane layer 31 may cover the first electrode 13 and the sensing layer 14 of the sensor 1. Thus, the first electrode 13 and the sensing layer 14 can be protected.

In some examples, the first membrane layer 31 may be made from a first solution, which may include a first-type polymer and an organic solvent. The first-type polymer may be polyurethane. In this case, the first membrane layer 31, including polyurethane, can limit the permeation of sterilizing gas when the sensor 1 undergoes gas sterilization, forming an isolation barrier to reduce the impact of sterilizing gas on the sensing layer 14, particularly the effect of gaseous ethylene oxide on the active substance in the sensing layer 14, enabling the sensor 1 to be sterilized using a gas sterilization method. Compared with the sensor 1 without first membrane layer 31, the reduction in sensitivity of the sensor 1 with first membrane layer 31 before and after sterilization is significantly reduced, and higher sensitivity can still be maintained after sterilization. Furthermore, the first membrane layer 31, including polyurethane exhibits good biocompatibility, improving the safety of the sensor 1 when implanted subcutaneously in a host; in addition, the first membrane layer 31 also has a permeation-limiting effect on a particular analyte (e.g., glucose), and can function as a semi-permeable membrane.

In some examples, the first membrane layer 31 may also be applied in other scenarios requiring limitation of ethylene oxide (EO) permeation, by disposing the first membrane layer 31 on a predetermined target. That is, the second aspect of the present disclosure may provide a first membrane layer 31 that is capable of limiting the permeation of sterilizing gas.

In some examples, the method of making the first membrane layer 31 may include: preparing a first solution; coating the first solution on a predetermined location; and forming the first membrane layer 31 after drying.

As previously mentioned, a third aspect of the present disclosure is directed to an analyte monitoring device 100.

In some examples, the analyte monitoring device 100 may include an electronic component 2 and a sensor 1 electrically connected to the electronic component 2 (see FIG. 1). Thus, the analyte can be monitored by the sensor 1. The sensor 1 described in the third aspect of the present disclosure is identical to the sensor 1 described in the first aspect of the present disclosure, and the specific structure and details thereof may be referred to in the description of the first aspect of the present disclosure and will not be described in detail here. That is, in some examples, the analyte monitoring device 100 may include an electronic component 2 and the sensor 1 as described in the first aspect of the present disclosure. Thus, a sensor 1 having a wider material selection range and broader functionality may be provided, and the analyte monitoring device 100 including the sensor 1 may meet the requirements of a wider range of application scenarios.

In particular, in some examples, the analyte monitoring device 100 may include a sensor 1 that is resistant to sterilizing gas. That is, in some examples, the analyte monitoring device 100 may include a sensor 1 having an isolation layer. In this case, the sensor 1 is resistant to sterilizing gas, enabling the sensor 1 to be assembled with the electronic component 2 for gas sterilization without performing radiation sterilization on the sensor 1 alone.

In some examples, the analyte monitoring device 100 may be assembled as a unit at the time of shipment, and the user need not perform the additional operation of assembling the electronic component 2 with the sensor 1 at the time of use. Thus, it is possible to facilitate user operation. It should be noted that although the sensor 1 provided by the present disclosure may be of an integral design with the electronic component 2, the two may also be provided as a detachable configuration, if desired. The "integral design" should not be construed as limiting the sensor 1 of the present disclosure.

In view of the foregoing, and in accordance with the present disclosure, a sensor 1 and an analyte monitoring device 100 can be provided having a wider material selection range. In particular, a sensor 1 and an analyte monitoring device 100 resistant to sterilizing gas can be provided.

To further illustrate the present disclosure, the sensor 1 and the first membrane layer 31 provided by the present disclosure are described in detail below with reference to embodiments in which the first membrane layer 31 serves as an isolation layer, and the beneficial effects achieved by the present disclosure are fully explained with reference to comparative examples.

It should be noted that in the embodiments and comparative examples of the present disclosure, the reagents and instruments used are commercially available products, unless otherwise specified.

First, a plurality of sensors, including a first electrode provided with a sensing layer containing glucose oxidase, are prepared for use.

### [Examples]

First, second, and third solutions (no third solution in some of the examples) were prepared according to Table 1-1 and Table 1-2. On the prepared sensors, the second solution was coated to form a second membrane layer, the first solution was coated to form a first membrane layer, and the third solution was coated to form a third membrane layer, thereby obtaining sensors having a polymer membrane in Examples 1 to 11. The PEO in Table 1-1 and Table 1-2 belongs to the hydrophilic group and soft segment, and PEGDGE refers to polyethylene glycol diglycidyl ether; PEO-containing polyurethane was taken as an example for the experiment.

**Table 1-1**

| | | First solution (isolation layer) | | Second solution (protective layer) | | Third solution (biocompatible layer) | | membrane layer thickness |
|---|---|---|---|---|---|---|---|---|
| | | Components | Concentration (mass fraction) | Components | Concentration (mg/mL) | Components | Concentration (mass fraction) | |
| Example 1 | Solute | Polyether-based polyurethane , containing 10% PEO | 3% | (Poly-4-vinylpyridine -g-polyethylene glycol)-co-polystyrene | 100 | \ | \ | Isolation layer 15 µm Protective layer 10 µm |
| | | | | PEGDGE | 15 | | | |
| | Solvent | Tetrahydrof uran | \ | 80% ethanol in water | \ | \ | \ | |
| Example 2 | Solute | Polyether-based polyurethane , containing 10% PEO | 3% | (Poly-4-vinylpyridine -g-polyethylene glycol)-co-polystyrene | 100 | \ | \ | Isolation layer 25 µm Protective layer 10 µm |
| | | | | PEGDGE | 15 | | | |
| | Solvent | Tetrahydrofuran | \ | 80% ethanol in water | \ | \ | \ | |
| Example 3 | Solute | Polyether-based polyurethane , containing 10% PEO | 3% | (Poly-4-vinylpyridine -g-polyethylene glycol)-co-polystyrene | 100 | \ | \ | Isolation layer 35 µm Protective layer 10 µm |
| | | | | PEGDGE | 15 | | | |
| | Solvent | Tetrahydrof uran | \ | 80% ethanol in water | \ | \ | \ | |
| Example 4 | Solute | Polyether-based polyurethane , containing 10% PEO | 10% | (Poly-4-vinylpyridine -g-polyethylene glycol)-co-polystyrene | 100 | \ | \ | Isolation layer 40 µm Protective layer 10 µm |
| | | | | PEGDGE | 15 | | | |
| | Solvent | Tetrahydrof uran | | 80% ethanol in water | \ | \ | \ | |
| Example 5 | Solute | Polyether-based polyurethane , containing 10% PEO | 5% | (Poly-4-vinylpyridine -g-polyethylene glycol)-co-polystyrene | 100 | \ | \ | Isolation layer 15 µm Protective layer 10 µm |
| | | | | PEGDGE | 15 | | | |
| | Solvent | Tetrahydrof uran | \ | 80% ethanol in water polyethylene glycol)-co-polystyrene | \ | \ | \ | |
| Example 6 | Solute | Polyether-based polyurethane , containing 10% PEO | 0.5% | (Poly-4-vinylpyridine -g-polyethylene glycol)-co-polystyrene | 100 | \ | \ | Isolation layer 15 µm Protective layer 10 µm |
| | | | | PEGDGE | 15 | | | |
| | Solvent | Tetrahydrof uran | \ | 80% ethanol in water | \ | \ | \ | |

**Table 1-2**

| | | First solution (isolation layer) | | Second solution (protective layer) | | Third solution (biocompatible layer) | | membrane layer thickness |
|---|---|---|---|---|---|---|---|---|
| | | Components | Concentrati on (mass fraction) | Components | Concentration (mg/mL) | Components | Concentrati on (mass fraction) | |
| Example 7 | Solute | Polyester-based polyurethane, containing 10% PEO | 3% | (Poly-4-vinylpyridin e-g-polyethylene glycol)-co-polystyrene | 100 | \ | \ | Isolation layer 15 µm Protective layer 10 µm |
| | | | | PEGDGE | 15 | \ | \ | |
| | Solve nt | Tetrahydrofur an | \ | 80% ethanol in water | \ | \ | \ | |
| Exampl e 8 | Solute | Polycarbonate -based polyurethane, containing 10% PEO | 3% | (Poly-4-vinylpyridin e-g-polyethylen e glycol)-co-polystyrene | 100 | \ | \ | Isolation layer 15 µm Protective layer 10 µm |
| | | | | PEGDGE | 15 | \ | \ | |
| | Solve nt | Tetrahydrofur an | \ | 80% ethanol in water | \ | \ | \ | |
| Exampl e 9 | Solute | Polyether-based polyurethane, containing 10% PEO | 3% | (Poly-4-vinylpyridin e-g-polyethylene glycol)-co-polystyrene | 100 | Polyether-based polyurethane, containing 30% PEO | 3% | Isolation layer 15 µm Protective layer 10 µm Biocompatib le layer 15 µm |
| | | | | PEGDGE | 15 | | | |
| | Solvent | Tetrahydrofuran | \ | 80% ethanol in water | \ | Tetrahydrofuran | \ | |
| Example 10 | Solute | Polyether-based polyurethane, containing 10% PEO | 3% | (Poly-4-vinylpyridin e-g-polyethylene glycol)-co-polystyrene | 100 | Polyether-based polyurethane, containing 30% carboxybetaine | 3% | Isolation layer 15 µm Protective layer 10 µm Biocompatible layer 15 µm |
| | | | | PEGDGE | 15 | | | |
| | Solvent | Tetrahydrofuran | \ | 80% ethanol in water | \ | Tetrahydrofuran | \ | |
| Example 11 | Solute | Polyether-based polyurethane, containing 10% PEO | 3% | (Poly-4-vinylpyridin e-g-polyethylene glycol)-co-polystyrene | 100 | Polyether-based polyurethane, containing 30% sulfobetaine | 3% | Isolation layer 15 µm Protective layer 10 µm Biocompatible layer 15 µm |
| | | | | PEGDGE | 15 | | | |
| | Solvent | Tetrahydrofuran | \ | 80% ethanol in water | \ | Tetrahydrofuran | \ | |

### [Comparative Example 1]

The commercially available continuous glucose monitoring system (Shenzhen Silicon-based Sensing Technology Co., Ltd., model: GS1) was selected as Comparative Example 1.

### [Comparative Example 2]

The sensor of Comparative Example 2 was prepared according to the same procedure as Example 1, with the difference from Example 1 being that: the second membrane layer (protective layer) was not provided.

### [Comparative Example 3]

The sensor of Comparative Example 3 was prepared according to the same procedure as Example 4, with the difference from Example 4 being that: the first membrane layer (isolation layer) had a thickness of 50 µm.

### [Comparative Example 4]

The sensor of Comparative Example 4 was prepared according to the same procedure as Example 1, with the difference from Example 1 being that the polyether-based polyurethane in the first solution contained 50% PEO.

### The performance of the sensor was tested:

### (1) Cytotoxicity test (biocompatibility test)

The sensors of Example 1 and Example 9 were taken as examples, and cytotoxicity tests were performed, specifically: a control group and an experimental group were designed, wherein the control group included a blank group (added with blank control cell culture medium), a negative control group (added with high-density polyethylene extract solution (non-toxic substance)), and a positive control group (added with latex glove extract solution (toxic substance)); the experimental group included the first solution of Example 1 and the third solution of Example 9 at different concentrations, seeing Table 2 for details; cells were inoculated into the experimental group and the control group, incubated, and then cell viability was measured; the cytotoxicity test results are shown in Table 2.

**Table 2**

| | Group | Average absorbance | Standard deviation | Cell relative viability |
|---|---|---|---|---|
| Control group | Blank group | 1.1896 | 0.0401 | 100.00% |
| | Negative control group | 1.1246 | 0.0228 | 94.54% |
| | Positive control group | 0.0077 | 0.0034 | 0.65% |
| Example 1 | 1-1 (100% extract solution) | 1.1108 | 0.0729 | 93.38% |
| | 1-2 (50% extract solution) | 1.1293 | 0.0299 | 94.93% |
| | 1-3 (25% extract solution) | 1.1509 | 0.0435 | 96.75% |
| | 1-4 (12.5% extract solution) | 1.2187 | 0.0553 | 102.45% |
| Example 9 | 9-1 (100% extract solution) | 1.1361 | 0.0451 | 95.50% |
| | 9-2 (50% extract solution) | 1.1546 | 0.0352 | 97.06% |
| | 9-3 (25% extract solution) | 1.1762 | 0.0518 | 98.87% |
| | 9-4 (12.5% extract solution) | 1.2072 | 0.0397 | 104.57% |

### (2) Sensitivity test

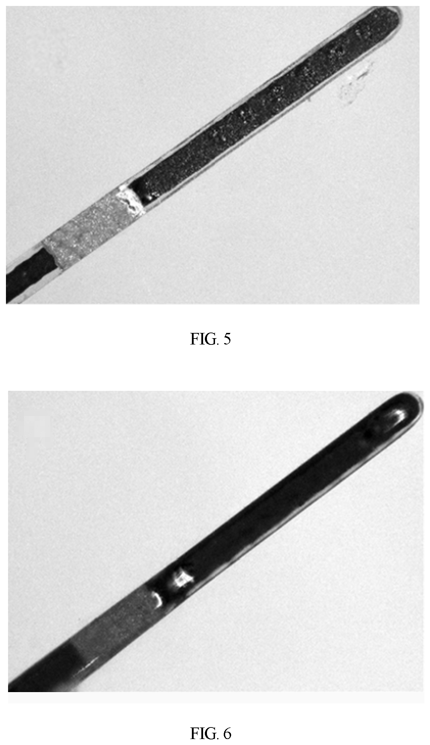

The sensors of each example and each comparative example were sterilized using ethylene oxide under the following sterilization conditions: temperature: 52 ± 3°C, humidity: 30-85%, action time: 360 min (minutes), and concentration: 600 ± 40 mg/L. In the drawings, FIG. 5 is a diagram showing the appearance of the sensor of Example 1 of the present disclosure after sterilization, and FIG. 6 is a diagram showing the appearance of the sensor of Comparative Example 1 of the present disclosure after sterilization.

The sensitivity of each sensor was tested, specifically: the sensor was exposed to standard phosphate buffered saline (PBS buffer) with different concentrations of glucose at 37°C ± 1°C, the response currents at different glucose concentrations were measured, and the response currents were fitted to obtain a standard curve, so that the sensitivity of each sensor could be calculated based on the fitted standard curve. The glucose concentrations were the following values: 2.2 mM (millimoles per liter), 5 mM, 10 mM, 15 mM, 20 mM, 25 mM. The measured sensitivities are shown in Table 3. In Table 3, the descending range = (sensitivity before sterilization - sensitivity after sterilization) / sensitivity before sterilization

× 100%; the greater the descending range (also referred to as attenuation range), the greater the influence by ethylene oxide, and the weaker the resistance of the sensor to ethylene oxide; Conversely, the smaller the descending range, the stronger the resistance of the sensor to ethylene oxide.

**Table 3**

| | Sensitivity before sterilization (nA/mM) | Sensitivity after sterilization (nA/mM) | Reduction range |
|---|---|---|---|
| Example 1 | 1.52 | 1.32 | 13.2% |
| Example 2 | 1.23 | 1.16 | 5.7% |
| Example 3 | 0.85 | 0.82 | 3.5% |
| Example 4 | 0.65 | 0.63 | 3.1% |
| Example 5 | 1.46 | 1.37 | 6.2% |
| Example 6 | 1.60 | 1.42 | 11.3% |
| Example 7 | 1.37 | 1.19 | 13.1% |
| Example 8 | 1.25 | 1.12 | 10.4% |
| Example 9 | 1.31 | 1.18 | 9.9% |
| Example 10 | 1.35 | 1.23 | 8.9% |
| Example 11 | 1.40 | 1.22 | 12.9% |
| Comparative Example 1 | 1.64 | 0.49 | 70.1% |
| Comparative Example 2 | / | / | / |
| Comparative Example 3 | / | / | / |
| Comparative Example 4 | 2.42 | 0.21 | 91.3% |

### Analysis of results

As can be seen from FIG. 5 and FIG. 6, the appearance of the sensor of Example 1 did not significantly change after sterilization, whereas the sensor of Comparative Example 1 changed and became noticeably green (it should be noted that, in order to comply with the provisions of *Guidelines for Patent Examination*, the drawings were converted to black and white in the present disclosure), indicating that the sensor of Comparative Example 1 without a first membrane layer was eroded after sterilization.

As can be seen from Table 3, the decrease in sensitivity of the sensors of each example before and after sterilization was small, indicating that the sensors of each example were resistant to ethylene oxide. However, the sensitivity of the sensors of the respective comparative examples decreased significantly after sterilization, and some could not detect any sensitivity. The following is a specific analysis:

In Comparative Example 1, the sensor of Comparative Example 1 could not be subjected to the gas sterilization using ethylene oxide because the commercially available product did not have such a first membrane layer as in the present disclosure, and the sensitivity of the sensor was greatly reduced after sterilization. In Comparative Example 2, since no second membrane layer was provided, the electrode was corroded after applying the first solution containing an organic solvent, resulting in undetectable sensitivity, indicating that the electrode can be effectively protected by the second membrane layer of the present disclosure. In Comparative Example 3, since the first membrane layer had a thickness of 50 µm, an excessive increase in membrane thickness resulted in excessive diffusion resistance of the first membrane layer to glucose, and effective sensitivity could not be detected. In Comparative Example 4, the content of PEO in the first membrane layer was 50%, namely, the content of soft segment and hydrophilic group was 50%; the overall hydrophilicity of the first membrane layer was relatively high, and the diffusion resistance to glucose was relatively small, so that the sensor had relatively high sensitivity before sterilization, but also resulted in poor isolation effect of the first membrane layer on ethylene oxide, and the sensitivity of the sensor greatly decreased after sterilization.

By comparing Example 1 with Examples 2-3, it can be seen that an increase in membrane thickness leads to a decrease in sensitivity, but also reduces the degree of sensitivity decay after sterilization, indicating that an increase in the membrane thickness of the first membrane layer can reduce the damage to the electrode caused by ethylene oxide sterilization.

By comparing Example 1 with Example 4, it can be seen that since the concentration and thickness of the polyether-based polyurethane in Example 4 are both greater than those in Example 1, the degree of sensitivity decay after sterilization is reduced, but the overall sensitivity of Example 4 is lower than that of Example 1.

By comparing Example 1 with Examples 5-6, it can be seen that Examples 5-6 use different concentrations of polyether-based polyurethane to prepare the first membrane layer, respectively. Due to the different solution concentrations, there are slight differences in sensitivity before sterilization and in the degree of sensitivity decay after sterilization, but no significant differences.

By comparing Example 1 with Examples 7-8, it can be seen that Examples 7-8 use different types of polyurethane, respectively. Since these two types of polyurethane are more hydrophobic than polyether-based polyurethane, the diffusion resistance of the first membrane layer to glucose is greater, and the sensitivity is reduced, but the degree of sensitivity decay after sterilization is also reduced accordingly, indicating that different types of polyurethane can each achieve an isolation effect.

By comparing Example 1 with Examples 9-11, it can be seen that Examples 9-11 are sensors with third membrane layers of different components, respectively. Due to the increased overall membrane thickness of the polymer membrane, the sensitivity of the sensors of Examples 9-11 is decreased compared with that of Example 1, but the degree of sensitivity decay after sterilization is also reduced accordingly, providing a stronger protective effect; and the third solution of Example 9 showed stronger biocompatibility in the cytotoxicity test than the first solution of Example 1, indicating that adding the third membrane layer helps improve the biocompatibility of the sensor.

In summary, the sensors of the various examples are highly resistant to ethylene oxide (i.e., sterilizing gas). In contrast, the sensors obtained in the comparative examples fail to achieve the performance and effects of the sensors obtained in the above-mentioned examples. By means of the protective membrane (second membrane layer) of the present disclosure, it is possible to reduce the adverse effects of harmful substances in the first membrane layer on the internal structure.

According to the present disclosure, it is possible to provide a biosensor with a wider material selection range, which can meet the needs of more application scenarios.

Although the present disclosure has been specifically described above with reference to the accompanying drawings and examples, it should be understood that the above description does not limit the present disclosure in any form. It will be apparent to those skilled in the art that various modifications and variations can be made to the present disclosure without departing from the spirit or scope of the disclosure, all of which fall within the scope of the present disclosure.

## Claims

1. A biosensor, wherein
the biosensor comprises a first electrode provided with a sensing layer, and a polymer membrane,
the polymer membrane covers at least the sensing layer and the first electrode, the polymer membrane comprises a first membrane layer and a second membrane layer,
the first membrane layer comprises a substance that can cause damage to the sensing layer and/or the first electrode upon direct contact, and the second membrane layer is closer to the first electrode than the first membrane layer to separate the first membrane layer from the sensing layer and the first electrode.

2. The biosensor according to claim 1, wherein
the first membrane layer is configured to reduce an effect of a sterilizing gas on the sensing layer when the biosensor is subjected to gas sterilization.

3. The biosensor according to claim 1 or 2, wherein
the first membrane layer is made from a first solution comprising a first-type polymer and a solvent.

4. The biosensor according to claim 3, wherein the first-type polymer is polyurethane.

5. The biosensor according to claim 4, wherein
the mass fraction of the first-type polymer in the first solution is from 0.1% to 15%.

6. The biosensor according to claim 3, wherein
a solvent of the first solution can cause damage to the sensing layer and/or the first electrode upon direct contact.

7. The biosensor according to claim 6, wherein
the solvent of the first solution is an organic solvent, the organic solvent is a mixed solvent of any one or more of tetrahydrofuran, dichloromethane, and N,N-dimethylacetamide.

8. The biosensor according to claim 1, wherein
the first membrane layer has a diffusion-limiting effect on an analyte sensed by the biosensor.

9. The biosensor according to claim 1, wherein
the first membrane layer has biocompatibility.

10. The biosensor according to claim 8 or 9, wherein
the first solution comprises a first-type polymer, the first-type polymer is polyurethane, the first-type polymer has a soft segment, and the proportion of the soft segment in the first-type polymer is from 5% to 50%.

11. The biosensor according to claim 8 or 9, wherein
the first solution comprises a first-type polymer, the first-type polymer is polyurethane, the first-type polymer has a hydrophilic group, and the proportion of the hydrophilic group in the first-type polymer is from 1% to 30%.

12. The biosensor according to claim 1, wherein
the first membrane layer has a thickness of 1 µm to 100 µm.

13. The biosensor according to claim 1, wherein
the substance in the second membrane layer does not react with the substance in the first membrane layer.

14. The biosensor according to claim 1 or 13, wherein
the second membrane layer comprises a second-type polymer, the second-type polymer is a zwitterionic polymer or a copolymer formed from neutral monomers.

15. The biosensor according to claim 1 or 13, wherein
the second membrane layer has a thickness of 1 µm to 30 µm.

16. The biosensor according to claim 1, wherein
the polymer membrane further comprises other functional membrane layers selected from any one or more of an anti-interference layer, a biocompatible layer, an antibacterial layer, a temperature-compensation layer, and a signal-enhancement layer.

17. The biosensor according to claim 1 or 2, wherein
the polymer membrane further comprises a third membrane layer, the third membrane layer is a biocompatible layer, the first membrane layer is located between the second membrane layer and the third membrane layer.

18. The biosensor according to claim 17, wherein
the biocompatible layer comprises a third-type polymer, the third-type polymer is a hydrophilic group-containing polyurethane or a cross-linked water-soluble polyurethane.

19. The biosensor according to claim 18, wherein
when the third-type polymer is the hydrophilic group-containing polyurethane, in the third-type polymer, the proportion of the hydrophilic group is from 20% to 50%.

20. The biosensor according to claim 17, wherein
the biocompatible layer has a thickness of 1 µm to 30 µm.

21. The biosensor according to claim 1, wherein,
the analyte sensed by the biosensor is one or more types.

22. The biosensor according to claim 1 or 21, wherein
the sensing layer comprises one or more of glucose oxidase, lactate oxidase, and hydroxybutyrate dehydrogenase.

23. The biosensor according to claim 1, wherein,
the biosensor further comprises a second electrode different from the first electrode, and the polymer membrane also covers the second electrode.

24. An analyte monitoring device, comprising an electronic component and the biosensor according to any one of claims 1 to 23, the electronic component being electrically connected to the biosensor.
